# EUROPEAN PATENT APPLICATION

(11) **EP 2 075 022 A1**
(43) Date of publication of application: **01.07.2009**
(21) Application number: 08171614.4
(22) Date of filing: 15.12.2008
(51) Int. Cl.: A61M 16/00

(54) **Color-coding system for breathing bags**

(30) Priority: 21.12.2007 US 4958
(71) Applicant: Medline Industries, Inc.,, Mundelein, IL 60060-4486 (US)
(72) Inventor: Finnigan, Timothy M., Libertyville, IL 60048 (US); Zettergren, Linda J., Antioch, IL 60022 (US)
(74) Representative: Schwarze, Holm

(57) **Abstract**

A breathing bag (10) is disclosed. The breathing bag comprises a bag body (12) enclosing an interior volume of the breathing bag. The bag body includes a distal end portion (22) and a neck portion (16) terminating in an open proximal end of the breathing bag. The breathing bag further comprises a bushing (18) coupled to the neck portion. The breathing bag includes at least one indicia thereon. The at least one indicia has a color corresponding with a size of the breathing bag.

## Description

### FIELD OF THE INVENTION

The present invention relates generally to breathing bags. More particularly, the present invention relates to breathing bags having indicia thereon for aiding or assisting in identifying the size and/or volume of the bags.

### BACKGROUND OF THE INVENTION

During surgical procedures, patients unable to breathe for themselves generally require the assistance of doctors, nurses, or skilled medical technicians. To prevent suffocation, a manually-operated breathing bag connected to an anesthesia machine and/or a breathing circuit is often used to force anesthesia, oxygen, nitrogen, other gases, or combinations thereof into a patient's lungs. A doctor, nurse, or medical technician may place a face mask directly over the patient's mouth and nose and connect the face mask to a breathing circuit. The breathing bag is then squeezed and oxygen and/or other gases are pushed into the patient's lungs. This is common practice, as the breathing bag is used to ventilate a patient who cannot breath on his or her own due to anesthesia.

Breathing bags are generally available in various sizes, including 0.5-liter, 1-liter, 2-liter, and 3-liter. Typically, the size of a breathing bag is not indicated anywhere on the breathing bag. The size of the breathing bag may be indicated only in, for example, the packaging in which the breathing bag is provided. When the breathing bag is in use, it has generally been unassociated with the packaging for quite some time. Therefore, a user must visually identify the size of the breathing bag. In their non-inflated state, the various sizes of breathing bags may appear to have generally the same or similar dimensions. Because of this, a particular size breathing bag may become easily mixed and/or confused with another size(s) breathing bag(s). Thus, visually identifying the size of a breathing bag is prone to error, which can be wasteful and/or dangerous.

Because the sizes of the breathing bags are not readily apparent from a visual inspection, it is often difficult and/or time-consuming to determine the size of the breathing bags. This difficulty in distinguishing size is prevalent, for example, in an operating room where healthcare professionals are often rushed and preoccupied and, thus, may not have the time required to definitively determine the size of a breathing bag being used in an anesthesia and/or breathing circuit. This is especially problematic when an adult-sized breathing bag is confused with a pediatric-sized breathing bag and vice versa. For example, when an adult-sized breathing bag is used for a pediatric patient, the breathing bag, which is generally too large for the pediatric patient, may force too much air into the pediatric patient's lungs. This can be harmful to the pediatric patient.

Therefore, there exists a need for an improved system of coding breathing bags that promotes efficiency in size determination.

### SUMMARY OF THE INVENTION

According to one embodiment, a breathing bag is disclosed. The breathing bag comprises a bag body enclosing an interior volume of the breathing bag. The bag body includes a distal end portion and a neck portion terminating in an open proximal end of the breathing bag. The breathing bag further comprises a bushing coupled to the neck portion. The breathing bag includes at least one indicia thereon. The at least one indicia has a color corresponding with a size of the breathing bag.

According to one process, a method of distinguishing a size of a breathing bag is disclosed. The method comprises the act of providing a breathing bag. The breathing bag includes a bag body enclosing an interior volume of the breathing bag. The bag body includes a distal end portion and a neck portion terminating in an open proximal end of the breathing bag and a bushing coupled to the neck portion. The method further comprises the act of providing at least one indicia on the breathing bag. The at least one indicia has a color corresponding with a size of the breathing bag. The method further comprises the act of
comparing the color of the at least one indicia to a color code. The color code has a color corresponding with a size of the breathing bag.

According to another process, a method of manufacturing a breathing bag is disclosed. The method comprises the act of forming a bag body. The bag body includes a distal end portion and a neck portion terminating in an open proximal end of the breathing bag. The method further comprises the act of coupling a bushing to the neck portion. The method further comprises the act of providing at least one indicia. The at least one indicia has a color corresponding with a size of the breathing bag.

According to another embodiment, a breathing bag is disclosed. The breathing bag comprises a bag body enclosing an interior volume of the breathing bag. The bag body includes a distal end portion and a neck portion terminating in an open proximal end of the breathing bag. The breathing bag further comprises a bushing coupled to the neck portion. The breathing bag further comprises at least one piece of tape coupling the bushing to the neck portion. The at least one piece of tape has at least one indicia thereon. The at least one indicia includes a color corresponding with a size of the breathing bag.

According to another process, a method of manufacturing a breathing bag is disclosed. The method comprises the act of forming a bag body. The bag body includes a distal end portion and a neck portion terminating in an open proximal end of the breathing bag. The method further comprises the act of providing at least one indicia positioned on the bag body. The at least one indicia being associated with a size of the breathing bag.

The above summary of the present invention is not intended to represent each embodiment or every aspect of the present invention. The detailed description and Figures will describe many of the embodiments and aspects of the present invention.

### BRIEF DESCRIPTION OF THE DRAWINGS

The foregoing and other advantages of the invention will become apparent upon reading the following detailed description and upon reference to the drawings.

FIG. 1 illustrates a perspective view of a breathing bag according to one embodiment of the present invention.

FIG. 2 illustrates a perspective view of the breathing bag of FIG. 1 after a bushing has been inserted into a neck portion.

FIG. 3 illustrates a perspective view of the breathing bag of FIGs. 1 and 2 having a colored tape positioned around the neck portion according to one embodiment.

FIG. 4 illustrates a perspective view of the breathing bag of FIGs. 1 and 2 having numerical indicia positioned around the neck portion according to another embodiment.

FIG. 5 illustrates a perspective view of the breathing bag of FIGs. 1 and 2 having indicia printed on the bag body according to yet another embodiment.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the spirit and scope of the invention.

### DESCRIPTION OF ILLUSTRATIVE EMBODIMENTS

The present invention is directed to breathing bags having indicia thereon for aiding or assisting in determining the sizes of the breathing bags.

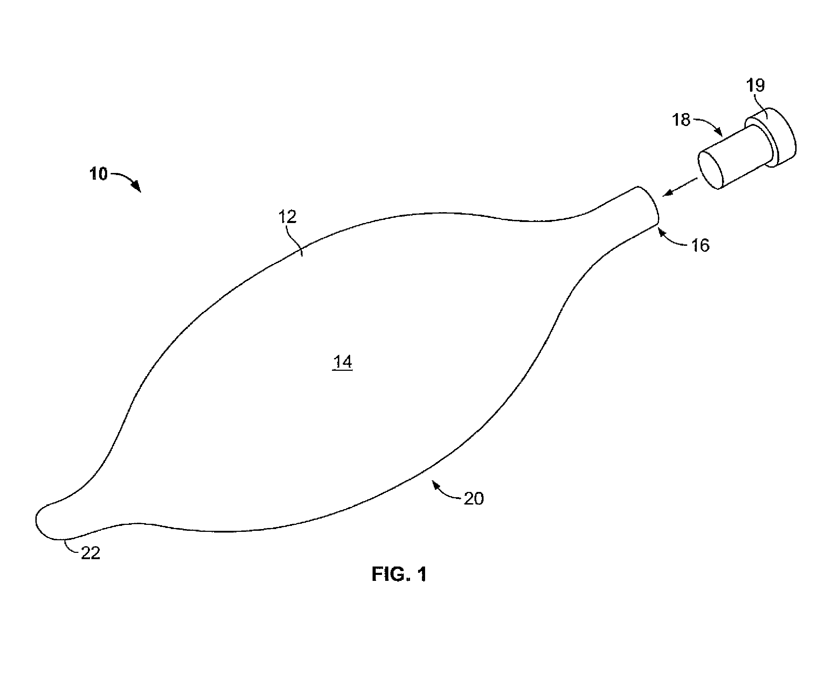

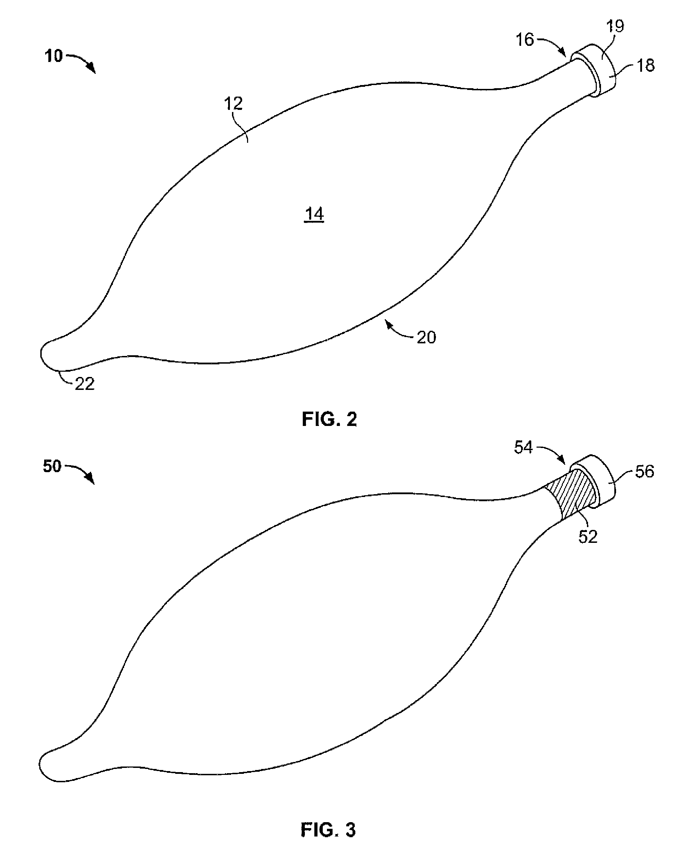

The structure of breathing bags (also known as reservoir bags or ventilator bags) is generally well-known. Referring to FIGs. 1, 2, for example, perspective views of a breathing bag 10 are illustrated according to one embodiment. The breathing bag 10 includes a bag body 12 having an interior volume and an exterior surface 14. The interior volume corresponds with a size (e.g., 0.5-liter, 1-liter, 2-liter, and 3-liter) of the breathing bag 10. One end of the breathing bag 10 further includes an open neck portion 16. Because the bag body 12 is generally relatively thin, the neck portion 16 may be insufficiently stiff or rigid to connect to a tubular or hollow cylindrical fitting of an anesthesia machine. Thus, the breathing bag 10 further includes a separate cylindrical bushing 18 inserted into the neck portion 16 to provide the required stiffness or rigidity to permit the breathing bag 10 to be connected to the anesthesia machine. The bushing 18 may be attached to the neck portion 16 using adhesive, heat sealing, or the like.

The bushing 18 may also include a flanged lip portion 19 that assists in securing the breathing bag 10 to an anesthesia and/or breathing circuit. The bushing 18 and/or the lip portion 19 may be formed of any suitable material including, but not limited to, polyvinyl chloride. The bushing 18 may have a thickness of about 0.125 inches (about 0.3 cm). The bushing 18 may also have other suitable thicknesses. Although the bushing 18 is sufficiently rigid to permit the breathing bag 10 to be connected to an anesthesia machine, the bushing 18 is sufficiently soft to permit the bushing 18 to slidably or wedgedly engage the anesthesia machine fitting. The bushing 18 may have an inwardly-tapered internal wall for being fitted to an inwardly-fitted anesthesia machine in a sliding or wedged air-tight engagement. Alternatively, the bushing 18 may have a straight or cylindrical internal wall for air-tight sliding or wedged engagement with a tubular or cylindrical anesthesia machine.

The breathing bag 10 of the illustrated embodiments has a generally elongated shape with an enlarged intermediate portion 20 tapering to the neck portion 16 and tapering to an opposing distal end 22. It is contemplated, however, that the breathing bag 10 may have other suitable shapes including, but not limited to circular, oval, dumbbell, S-shape, or the like.

The breathing bag 10 may be pulmonarily sized, i.e., sized for use in an anesthesia and/or breathing circuit, with a volume corresponding with the pulmonary volume of a patient. It is desirable to select a breathing bag having a volume similar to that of the lung of a patient with which the breathing bag is to be used so that the correct amount of air may be pumped into the patient's lung(s). The pulmonary volume may range from about 0.25 liters to about 5 liters. Thus, the interior volume enclosed by the breathing bag 10 may range from about 0.25 liters to about 5 liters. In some embodiments, the volume of the breathing bag 10 is about 0.5 liters to about 3 liters. Other volumes may also be enclosed by the breathing bag 10.

The breathing bag 10 may be formed of a latex material, a non-latex material, and/or a combination thereof. Non-limiting examples of non-latex materials that may be used to form the breathing bag 10 include neoprene, polyurethane, styrene-isoprene-styrene and/or styrene-butadiene-styrene compositions, polyvinylchloride (PVC), urethane, polyester elastomers, polyamide elastomers, olefinic polymers such as polypropylene or polyethylene, metalocene polymers, combinations thereof, and the like. The material used to form the breathing bag 10 may be in the form of a blown film, extruded sheet, solvent cast film, or other suitable web stock formed of the material. The thickness of the breathing bag 10 generally ranges from about 1 mil (about 0.02 mm) to about 25 mil (about 0.65 mm). It is contemplated, however, that the breathing bag 10 may have other thicknesses.

The exterior surface 14 of the breathing bag 10 may include a matte finish to ensure grippability of the breathing bag 10 when in use by a doctor, nurse, or medical technician engaged in manual compression or decompression of the breathing bag 10. The grippability of the breathing bag 10 is also beneficial for use with automated compression or expansion control means.

The characteristics, qualities, dimensions, and the like of the breathing bags of the embodiments of the present invention have been described with respect to the breathing bag 10 of FIGs. 1 and 2. However, it is contemplated that any of the breathing bags described herein (e.g., breathing bags 50, 70, 102 of FIGs. 3, 4, 5, respectively) may have any of the characteristics, qualities, dimensions, or the like described above.

Referring to FIG. 3, a breathing bag 50 is shown according to one embodiment. The breathing bag 50 of FIG. 3 is similar to the breathing bag 10 of FIGs. 1 and 2. The breathing bag 50, however, further includes colored indicia for indicating the size of the breathing bag 50. In the illustrated embodiment, the indicia includes colored tape 52 wrapped around a neck portion 54 of the breathing bag 50. The colored tape 52 may serve a functional purpose such as securing a bushing 56 to the neck portion 54. The colored tape 52 serves another purpose of providing a visual indication of the size of the breathing bag 50. Different tape colors may be used to indicate different sizes of breathing bags. For example, the colored tape on size 1-liter breathing bags may be white, the colored tape on size 2-liter breathing bags may be green, the colored tape on size 3-liter breathing bags may be blue, etc. The colored tape 52 may be readily compared to a color code or color chart to determine which size breathing bag 50 corresponds with a particular color. It is contemplated that the colored tape 52 may also be positioned elsewhere on the breathing bag 50.

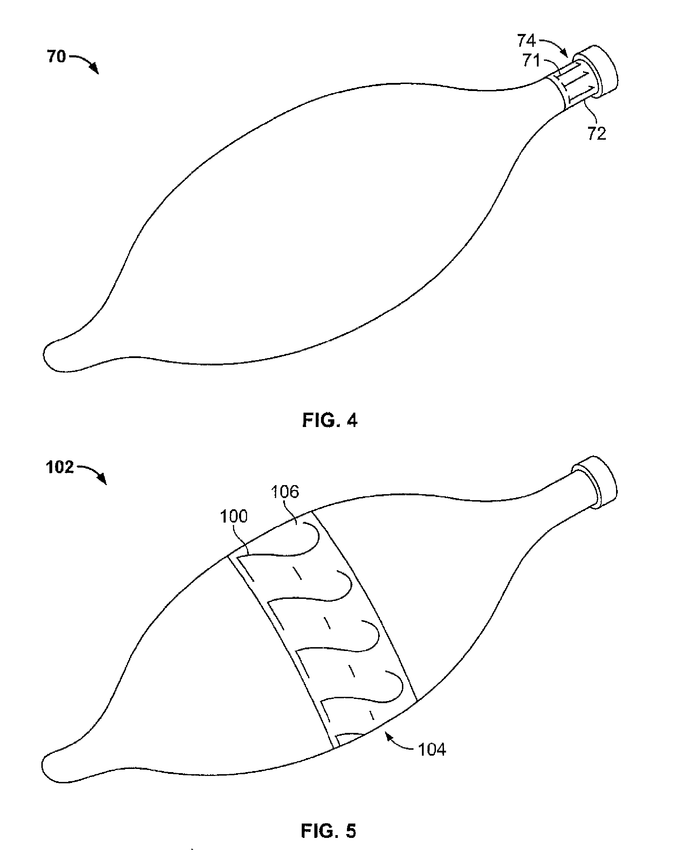

Additionally or alternatively, the breathing bags of the embodiments described herein may include other indicia such as, for example, at least one numerical indicia corresponding to the size of the breathing bag to assist in identifying the size of the breathing bag. The at least one numerical indicia may, for example, be a repeating number pattern. As shown, for example, in FIG. 4, a size 1-liter breathing bag 70 includes a repeating number pattern of the number "1" 71 positioned on tape 72 wrapped around a neck portion 74. Size 2-liter breathing bags may have a repeating pattern of the number "2", size 3-liter breathing bags may have a repeating pattern of the number "3", etc. It is contemplated that non-repeating indicia may also be used. It is also contemplated that other indicia may be used to indicate the size of the breathing bag 70 including, but not limited to, a repeating "ONE" indicia, a repeating "1L" indicia, or the like. Furthermore, the indicia may be positioned elsewhere on the breathing bag 70 besides on a tape 72 or on the neck portion 74. The location of the indicia may depend, for example, on customer preference.

The numerical indicia or other indicia illustrated and described above with respect to FIG. 4 may also be color-coded. Thus, for example, a number "1" on a size 1-liter breathing bag may be black, a number "2" on a size 2-liter breathing bag may be green, a number "3" on a size 3-liter breathing bag may be blue, etc. Using colored numerical indicia corresponding with the size of a breathing bag may be beneficial because the step of matching the color to a color code or chart to determine which size of breathing bag corresponds with a particular color would be eliminated. Furthermore, providing numerical or other indicia in addition to the color-coded indicia provides an extra safeguard for verifying that a particular color corresponds with the desired size by a visual indication of the numerical indicia on the breathing bag. Thus, the waste and/or danger associated with using an incorrect bag size may be minimized or eliminated.

It is contemplated that the indicia may be attached or positioned in areas of the breathing bag 10 other than those shown in the illustrated embodiments. It is further contemplated that any suitable means for applying and/or attaching the indicia to and/or forming the indicia on the breathing bag may also be used.

In one embodiment, the material from which the breathing bag or a portion thereof is made includes dye or other pigment additives to impart a desired color to the breathing bag or a portion thereof. The color of the dye or other pigment additives corresponds with the size of the breathing bag. Thus, referring to FIGs. 1 and 2, the entire bag body 12 of the breathing bag 10 may have a color corresponding to the size of the breathing bag 10.

In another embodiment, colored indicia indicating a size of a breathing bag may be printed (e.g., screen printed) directly onto the breathing bag. In FIG. 5, for example, a repeating numerical indicia 100 corresponding with the size (i.e., size 2-liter) of a breathing bag 102 is printed along or near an enlarged intermediate portion 104 of the breathing bag 102. The indicia 100 or an area surrounding the indicia (e.g., band 106) may have a color corresponding with the size of the breathing bag 102. Thus, it is contemplated that the colored indicia may of any suitable form positioned in any suitable location on the breathing bags of the embodiments described herein.

In yet another embodiment, the bushing (e.g., bushing 18 of FIGs. 1, 2) is color-coded to correspond with the size of the breathing bag 10. It is also contemplated that a portion of the bushing 18 (e.g., the lip 19) may be color-coded.

With the indicia described herein, one may readily identify the indicia and/or color of the indicia in order to identify the size of the breathing bag. In embodiments where the indicia is a color corresponding with a size of the breathing bag, the size may be identified by comparing the color of the indicia to a color code or chart that indicates which color corresponds to a particular size. The color code may also be committed to memory. The indicia of the embodiments described herein provide a highly visible and immediately recognizable means of identifying the different sizes of breathing bags. Moreover, the indicia described herein can generally be seen from a distance.

While the present invention has been described with reference to one or more particular embodiments, those skilled in the art will recognize that many changes may be made thereto without departing from the spirit and scope of the present invention. Each of these embodiments and obvious variations thereof is contemplated as falling within the spirit and scope of the invention, which is set forth in the following claims.

## Claims

1. A breathing bag comprising:
a bag body enclosing an interior volume of the breathing bag, the bag body including a distal end portion and a neck portion terminating in an open proximal end of the breathing bag; and
a bushing coupled to the neck portion,
wherein the breathing bag includes at least one indicia thereon, the at least one indicia corresponding with a size of the breathing bag.

2. The breathing bag of claim 1, wherein the at least one indicia is provided on the neck portion.

3. The breathing bag of claim 1, wherein the at least one indicia is colored tape positioned on the neck portion.

4. The breathing bag of claim 3, wherein the colored tape is adapted to couple the bushing to the neck portion.

5. The breathing bag of claim 1, wherein the at least one indicia is a color of at least a portion of the bag body.

6. The breathing bag of claim 1, wherein the at least one indicia includes at least one number corresponding to the size of the breathing bag.

7. The breathing bag of claim 1, wherein the at least one indicia is a color of the bushing or a portion thereof.

8. A method of distinguishing a size of a breathing bag, the method comprising the acts of:
providing a breathing bag, the breathing bag including a bag body enclosing an interior volume of the breathing bag, the bag body including a distal end portion and a neck portion terminating in an open proximal end of the breathing bag and a bushing coupled to the neck portion;
providing at least one indicia on the breathing bag, the at least one indicia having a color corresponding with a size of the breathing bag; and
comparing the color of the at least one indicia to a color code, the color code having a color corresponding with a size of the breathing bag.

9. The method of claim 8, wherein the at least one indicia is provided on the neck portion.

10. The method of claim 8, wherein the at least one indicia is colored tape positioned on the neck portion.

11. The method of claim 10, wherein the colored tape couples the bushing to the neck portion.

12. The method of claim 8, wherein the at least one indicia is a color of at least a portion of the bag body.

13. The method of claim 8, wherein the at least one indicia includes at least one number corresponding to the size of the breathing bag.

14. A method of manufacturing a breathing bag comprising the acts of:
forming a bag body, the bag body including a distal end portion and a neck portion terminating in an open proximal end of the breathing bag;
coupling a bushing to the neck portion; and
providing at least one indicia, the at least one indicia having a color corresponding with a size of the breathing bag.

15. The method of claim 14 further comprising attaching the bushing to the neck portion using at least one piece of tape, wherein the at least one indicia is at least one color located on the at least one piece of tape.

16. The method of claim 14, wherein the at least one indicia includes a pigment or dye used to form at least a portion of the bag body.

17. The method of claim 14 further comprising printing the at least one indicia on a portion of the bag body.
